Europäisches Patentamt

European Patent Office

Office européen des brevets

(11) Publication number: 0 362 755 A2

# EUROPEAN PATENT APPLICATION

(21) Application number: 89118209.9

(22) Date of filing: 02.10.89

(51) Int. Cl.5: C12N 5/00 , C12P 21/08 , C07K 15/00 , G01N 33/576 , G01N 33/577 , C12Q 1/70

(30) Priority: 03.10.88 JP 247479/88

(43) Date of publication of application: 11.04.90 Bulletin 90/15

(84) Designated Contracting States: BE CH DE FR GB IT LI NL

(71) Applicant: Asahi Kasei Kogyo Kabushiki Kaisha
2-6, Dojimahama 1-chome Kita-ku
Osaka-shi Osaka 530(JP)

(72) Inventor: Imawari, Michio
30-8-207 Motoyoyogi-cho
Shibuya-ku Tokyo(JP)
Inventor: Kaieda, Takeji
168-52 Obuchi
Fuji-shi Shizuoka(JP)

(74) Representative: Boeters, Hans Dietrich, Dr. et al
Boeters & Bauer, Patentanwälte
Thomas-Wimmer-Ring 14
D-8000 München 22(DE)

(54) Human cytotoxic t-cell clone and antibody thereto.

(57) A human cytotoxic T-cell clone with significant cytotoxicity against hepatocytes from patients with type non-A non-B virus hepatitis but not against hepatocytes from patients with type B virus hepatitis and an antibody against this cell clone. The human cytotoxic T-cell clone and antibody threto according to the present invention can contribute to the diagnosis, prophylaxis and treatment of type non-A non-B hepatitis.

Fig. 1

## HUMAN CYTOTOXIC T-CELL CLONE AND ANTIBODY THERETO

### FIELD OF THE INVENTION

The present invention relates to a human cytotoxic T-cell clone and an antibody against this cell clone, which can contribute to the diagnosis, prophylaxis and treatment of type non-A non-B hepatitis.

### BACKGROUND OF THE INVENTION

Data indicate that posttransfusion hepatitis currently occurs in about 10% of recipients of blood and that such hepatitis is caused in most cases (90% or more) by type non-A non-B virus, which is neither hepatitis A virus nor hepatitis B virus. Intensive research has so far been made in vain to reveal the nature of hepatitis non-A non-B virus.

Consequently, the current diagnosis of type non-A non-B hepatitis is based not on the detection of the virus or a virus-associated antibody but on the principle of exclusion. Thus, the diagnosis of type non-A non-B virus hepatitis is made based on the exclusion of other factors causative of liver dysfunction, such as hepatitis A, hepatitis B, cytomegalo and Epstein-Barr viruses (identifiable by serological tests), drugs including alcohol (deducible from the patient's history, autoimmune reactions and metabolic liver diseases. At present, no specific serological test for type non-A non-B hepatitis is available.

### SUMMARY OF THE INVENTION

In view of the above mentioned problem in the diagnosis of type non-A non-B hepatitis, the present inventors made intensive investigations in an attempt to establish a specific and highly reliable method of diagnosis for such hepatitis. As a result, they have succeeded in establishing, from peripheral lymphocytes obtained from a patient with chronic type non-A non-B hepatitis and a history of blood transfusion, a cytotoxic T-cell clone whose cytotoxicity can be significantly detected against hepatocytes from patients with type non-A non-B virus hepatitis but cannot be detected against hepatocytes from patients with type B virus hepatitis. This specificity indicates that the human cytotoxic T-cell clone seems to recognize a type non-A non-B virus-associated antigen.

This cytotoxic T-cell clone can be readily grown in cell culture. The clone exhibited significant cytotoxic activity against hepatocytes from a total of 15 patients with type non-A non-B hepatitis whereas it did not show cytotoxicity against hepatocytes from 9 patients with type B hepatitis. It was thus found that the diagnosis of type non-A non-B hepatitis can be made by using this human cytotoxic T-cell clone and assaying its cytotoxic activity against hepatocytes from hepatitis patients.

Furthermore, it was found that the diagnosis of type non-A non-B virus hepatitis can be made by using an antibody against an antigen binding site of T-cell receptor (antiidiotyic antibody) of this human cytotoxic T-cell clone. Thus, peripheral blood lymphocytes from patients with type non-A non-B hepatitis, when tested by the immunofluorescence and flow cytometry techniques using such an antiidiotypic antibody, showed a distinct fluorescence intensity as compared with peripheral blood lymphocytes from healthy donors. It was thus found that a human cytotoxic T-cell clone capable of specifically recognizing hepatocytes from type non-A non-B hepatitis patients and an antibody to the cell clone can be established or produced and can be used for establishing a highly reliable method of making a definite diagnosis of type non-A non-B hepatitis. The present invention has been completed based on these findings.

The invention thus provides a human cytotoxic T-cell clone with significant cytotoxicity against hepatocytes from patients with type non-A non-B virus hepatitis but not against hepatocytes from patients with type B virus hepatitis and, further, to an antibody to this human cytotoxic T-cell clone.

The invention also provides a method of diagnosing type non-A non-B hepatitis which comprises using the above-mentioned cytotoxic T-cell clone or the above-mentioned antibody.

### BRIEF DESCRIPTION OF THE DRAWINGS

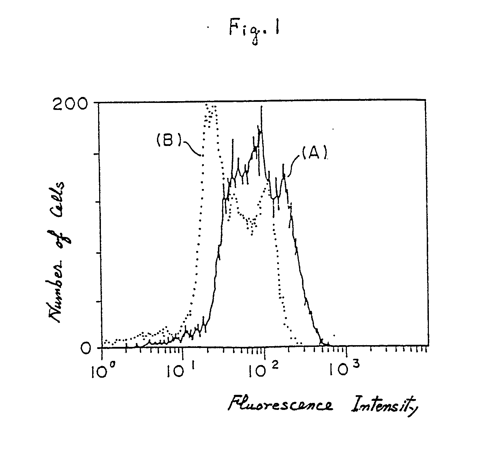

Fig. 1 graphically shows the difference between the reactivity of an antiidiotypic serum for lymphocytes (A) from a patient with type non-A non-B hepatitis and the reactivity of this antibody for lymphocytes (B) from a healthy donor.

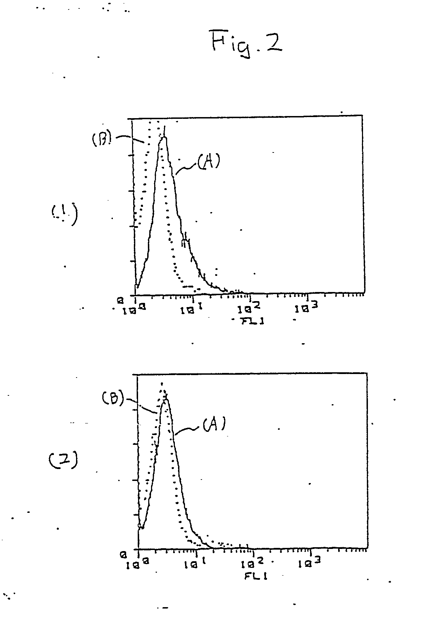

Fig. 2 graphically shows the specificity feature of the antiidiotypic monoclonal antibody to the human cytotoxic T-cell clone according to the invention. Fig. 2-(1) shows the reactivity (A) of this monoclonal antiboy for this clone in comparison with the reactivity (B) of a mouse antibody for the same clone. Fig. 2-(2) shows the reactivity (A) of this monoclonal antibody for a tumor-related antigen-recognizing human cytotoxic T-cell clone in comparison with the reactivity (B) of the mouse antibody for the clone.

## DETAILED DESCRIPTION OF THE INVENTION

The human cytotoxic T-cell clone according to the invention should desirably be prepared from peripheral blood lymphocytes obtained from a patient whose disease has been identified as type non-A non-B hepatitis by a conventional diagnostic method. Thus, peripheral blood is collected from such a patient, lymphocytes are separated from the blood by the method described in detail in Example 1 and are suspended in an interleukin-2-containing medium, and the suspension is distributed into the wells of each microplate. The plate is incubated in a carbon dioxide atmosphere. A number of T-cell clones are thus obtained. They are screened by the method described in detail in Example 2 to give one or more cytotoxic T-cell clones capable of specifically killing, or lysing, hepatocytes from patients with type non-A non-B hepatitis. Cells necessary for diagnostic purposes can be readily obtained by culturing such cytotoxic T-cell clones by the method described in detail in Example 3.

While a representative example of the thus-obtained cytotoxic T-cell clones specifically lysing hepatocytes from patients with type non-A non-B hepatitis, namely clone TA-NB-2 shown in Example 4, has been deposited, the scope of the invention is by no means limited to this deposited cytotoxic T-cell clone since the cytotoxic T-cell clone according to the invention can be obtained with good reproducibility by using the above-mentioned methods. As shown in Example 4, this representative cytotoxic T-cell clone exhibited significant cytotoxic activity against hepatocytes from all of 15 type non-A non-B hepatitis patients tested while, as shown in Comparative Example 1, it did not show any significant cytotoxic activity against hepatocytes from 9 patients with type B hepatitis. Thus, the invention provides a novel, epoch-making means for the diagnosis of type non-A non-B hepatitis.

For the production of an antibody to the cytotoxic T-cell clone according to the invention, an animal, such as a mouse, rat or rabbit, is immunized with the human cytotoxic T-cell clone and an antiserum is obtained. The antiserum prepared by the method described in detail in Example 5 is absorbed extensively with peripheral blood lymphocytes from a healthy donor or T-lymphoma cell line to give an antiserum containing an antibody against an antigen-binding site of T-cell receptor (antiidiotypic antibody) of the cytotoxic T-cell clone. Such generally known lymphoma cell lines as CEM and Molt-3 may be used for the absorption procedure. As shown in Example 7, it is also possible to obtain an antiidiotypic monoclonal antibody by immunizing a mouse(BALB/c) with the cytotoxic T-cell clone, performing somatic fusion of splenocytes from the immunized mouse with P3U1 (more precisely, P3-X63-Ag8-U1) mouse myeloma cells using polyethylene glycol (PEG) and screening a number of hybridoma strains obtained to select one or more hybridoma strains producing a clone-specific monoclonal antibody reactive only with this cytotoxic T-cell clone and nonreactive with other cytotoxic T-cell clones differing in specificity from this clone. Such and other monoclonal antibody production methods are described in detail monographs such as Hybridoma Techniques and Monoclonal Antibodies, edited by Takeshi Watanabe, R&D Planning, 1982.

A most general method for the diagnosis of hepatitis using the cytotoxic T-cell clone according to the invention is described below by way of example.

A liver biopsy specimen taken from a patient with suspected type non-A non-B hepatitis is minced using needles in a culture medium for separating hepatocytes. The resulting cell suspension is adjusted to a cell concentration of $3 \times 10^4$ per milliliter, distributed in 10-$\mu$l portions into wells of a microcytotoxicity Terasaki plate and incubated in an atmosphere of 95% air and 5% carbon dioxide for 24 hours. Thus, about 100 hepatocytes per well are caused to adhere to the plate. Ten microliters of a medium containing $1 \times 10^5$ cells/ml of the cytotoxic T-cell clone according to the invention is added to each well and incubated in an atmosphere of 95% air and 5% carbon dioxide for 18 to 24 hours. Each well is washed with physiological saline for detachment of those hepatocytes that have been killed by the cytotoxic T-cell clone and, then, adherent survivor hepatocytes are fixed with methanol, stained and counted. The percent kill is then calculated (cytotoxic activity). When the percent kill is 12% or more, the cytotoxicity is regarded to be statistically significant in view of the accuracy of the cytotoxic activity assay method employed. Thus, when the percent kill is below 12%, the result is judged negative and, when the percent kill is 12% or more, the result is judged positive.

A most general method for the diagnosis of hepatitis which uses the antibody to the cytotoxic T-cell clone in accordance with the invention is described below by way of example. Peripheral blood lymphocytes from a patient with suspected type non-A non-B hepatitis are suspended in a medium at a cell concentration of $1 \times 10^6$ cells/ml, an appropriate quantity of the antibody or an antiserum containing the antibody is added to the cell suspension, and the reaction is allowed to proceed at 4°C for 1 hour. After addition of physiological saline, the supernatant is removed by centrifugation, a commercially available fluorescein conjugated second antibody is added and the reaction is allowed to proceed at 4°C for 1 hour. Physiological saline is added and the supernatant is removed by centrifugation. The patient's lymphocytes thus prepared are subjected to flow cytometry and whether there are lymphocytes reacting with this antibody is judged. If there are lymphocytes reacting with said antibody, the result is positive. Detailed description of the techniques of flow cytometry can be found in a monograph such as Flow Cytometry, Techniques and Practice, edited by Kazuo Ohta, Kani Shobo, Tokyo, (1984).

As the other methods for the diagnosis of hepatitis which uses the antibody, for example, the method comprising reacting the antibody bonded to carriers such as micro plate or plastic bead with anti non-A non-B virus antibody present in the test serum, and then determining the resulting complex with an anti human IgG antibody can be employed.

In view of its specificity as shown in Example 4 and Comparative Example 1, it is considered that the human cytotoxic T-cell clone according to the invention quite possibly recognizes a type non-A non-B hepatitis virus-associated antigen occurring on the hepatocytes of patients with type non-A non-B hepatitis. Therefore, the antibody against the antigen-binding site of T-cell receptor of the cytotoxic T-cell clone according to the invention presumably has an antigen-binding site structure which may possibly serve as a model for a type non-A non-B hepatitis virus-associated antigen. Accordingly, if an antibody against the antigen-binding site of the above antibody could be obtained, this antibody would be an antibody capable of directly recognizing a type non-A non-B hepatitis virus-associated antigen and would be usable for the detection of such virus-associated antigen. In view of the current state of monoclonal antibody production technology, it is quite possible and realizable to produce an antibody to such type non-A non-B hepatitis virus-associated antigen in accordance with the above. It is to be noted that such antibody also falls within the scope of the present invention.

The antibody against the antigen-binding site of T-cell receptor for the cytotoxic T-cell clone according to the invention may possibly be used as a vaccine for enhancing the immunity against type non-A non-B hepatitis virus in vivo since this antibody has the ability to stimulate and activate the cytotoxic T-cell clone.

The dose of the vaccine for the prophylaxis and treatment of type non-A non-B hepatitis in the present invention may be generally in the range of 1 to 5 mg per body in an intramuscular injection. The administration may be carried out 2 or 3 times at intervals of one or two months.

The human cytotoxic T-cell clone and the antibody to the T-cell clone according to the invention provide highly reliable means for definite diagnosis of type non-A non-B hepatitis for which no specific diagnostic method is so far available.

The following examples illustrate the invention in further detail. Unless otherwise indicated, all parts, percents, ratios and the like are by weight.

EXAMPLE 1

Establishment of human cytotoxic T-cell clone specific to hepatocytes from patients with type non-A non-B hepatitis

(1) Separation of Peripheral Blood Lymphocytes from Patient

To 30 ml of blood (heparinized) taken from a patient with type non-A non-B hepatitis was added 30 ml of Hanks' balanced salt solution (Flow Laboratories Inc., U.S.A.). The mixture was distributed in 12-ml portions into 15-ml tubes (Corning 25310, Corning, U.S.A.) containing 3 ml of Ficoll-Paque (Pharmacia, Sweden) and centrifuged (1,600 rpm, 30 minutes). The interfacial lymphocyte layers were collected in a 15-ml tube, 10 ml of Hanks' solution was added, and the mixture was centrifuged (800 rpm, 10 minutes). The supernatant was removed, 10 ml of Hanks' solution was added, and the cell pellet was broken up thoroughly with tweezers. After further centrifugation (800 rpm, 10 minutes), the supernatant was removed, 4 ml of RPMI 1640 medium (Flow Laboratories, Inc., U.S.A.) supplemented with 20% fetal calf serum (Flow Laboratories, Inc., U.S.A.) was added to the sediment. After thorough mixing, a lymphocyte suspension ($1 \times 10^7$ cells/ml) was obtained.

(2) Cloning

A portion (0.5 ml) of the above lymphocytes suspension was set aside for cloning purposes. To the remainder (3.5 ml) was added 0.3 ml of 1 mg/ml mitomycin C (Kyowa Hakko Kogyo, Tokyo). The mixture was incubated at 37°C for 45 minutes, 10 ml of Hanks' solution was then added and, after thorough mixing, the mixture was centrifuged (800 rpm, 10 minutes). The supernatant was discarded, 10 ml of Hanks' solution was again added to the sediment and the resulting mixture was centrifuged (800 rpm, 10 minutes). The supernatant was removed, 10 ml of Hanks' solution was added, centrifugation was performed (800 rpm, 10 minutes), and the supernatant was removed. To the sediment was added 3 ml of RPMI 1640 medium supplemented with 20% fetal calf serum. Thorough mixing gave a feeder cells suspension ($1 \times 10^7$ cells/ml). Separately, 100 ml of RPMI 1640 medium supplemented with 20% fetal calf serum and containing 10% interleukin 2 (IL-2) (Boehringer Mannheim, West Germany) (IL-2 medium) was prepared. To this IL-2 medium were added 3 ml of the above-mentioned feeder cells suspension and 500 lymphocytes for cloning. After thorough mixing, 100 $\mu$l of PHA-P (phytohemagglutinin P; E-Y Laboratories, U.S.A.) was added. After thorough mixing, the mixture was distributed in 100-$\mu$l portions into wells of ten 96-well plates (Corning 25870) and incubated in a carbon dioxide incubator (37°C, 5% $CO_2$) for 2 weeks.

After the 2-week period, the ten plates were examined microscopically. Cell growth was observed in about 10% of the wells. In this way, about 300 T-cell clones were obtained. (In view of the percentage of the wells in which cell growth occurred, the cells in each of such wells can be considered to have proliferated from one lymphocyte. The lymphocytes that can grow under the conditions mentioned above are mostly T-cells. Therefore, the cells grown in each of the wells can be regarded as a T-cell clone.)

EXAMPLE 2

Microcytotoxicity assay

(1) Plate Preparation for Cytotoxic Activity Assay

A liver biopsy specimen (3 to 5 mm long) from a patient with hepatitis of type non-A non-B, type B or unknown type was transferred to a Petri dish containing 3 ml of RPMI 1640 medium containing 10% fetal calf serum and 5 mM EDTA and hepatocytes were mechanically detached from the tissue with two injection needles. The hepatocyte-containing medium was transferred to a 15-ml tube and centrifuged (600 rpm, 7 minutes). The supernatant was removed, 5 ml of the above medium was added to the sediment, the cell pellet was broken up using a pipet, followed by centrifugation (600 rpm, 7 minutes). The supernatant was removed, the above medium was again added and the same procedure was followed once more. To the sediment was added 1 ml of RPMI 1640 medium supplemented with 10% fetal calf serum. The hepatocyte suspension ($3 \times 10^4$ cells/ml) obtained after mixing was distributed in 10-$\mu$l (300 cells) portions into wells of a microcytotoxicity Terasaki plate (Nunc) and incubated at 37°C in an atmosphere of 95% air and 5% carbon dioxide to allow hepatocytes to adhere to the plate to give a plate for cytotoxic activity assay.

(2) Cytotoxic Activity Assay

A lymphocyte suspension containing the cytotoxic T-cell clone at a concentration of $1 \times 10^5$ cells/ml in RPMI 1640 medium supplemented with 10% fetal calf serum was distributed in 10-$\mu$l portions into 10 wells.

As a control, the medium alone was distributed in 10-$\mu$l portions into 10 wells. The Terasaki plate was incubated at 37°C in an atmosphere of 95% air and 5% carbon dioxide to allow the cytotoxic reaction to proceed. After 18 to 24 hours, those hepatocytes that had been killed were detached by gentle washing of the wells with physiological saline. The survivor hepatocytes were fixed by addition of 10 $\mu$l of methanol to each well. After removal of the methanol, 10 $\mu$l of a 1% eosin solution was added to each well for staining the survivor hepatocytes. The cells stained were counted and the cytotoxic activity (%) was calculated as follows:

$$\textbf{Cytotoxic Activity (\%)} = \frac{NC - NL}{NC} \times 100$$

where NC is the mean number of hepatocytes in the control wells and NL is the mean number of hepatocytes in the test wells with the cytotoxic T-cell clone added thereto.

## EXAMPLE 3

Cultivation of cytotoxic T-cell clone

Since the cytotoxic T-cell clone requires interleukin 2 (IL-2) for its growth, the clone was cultured in an IL-2-containing medium. The medium used was RPMI 1640 medium supplemented with 20% fetal calf serum and containing 10% IL-2 (Boehringer Mannheim). The initial cell density was $1 \times 10^5$ cells/ml and the final cell density was 1 to $2 \times 10^6$ cells/ml. For the cultivation, a 96-well plate (Corning 25870, capacity 200 $\mu$l per well), a 24-well plate (Corning 25820, capacity 2 ml per well) and culture bottles (Corning 25100, 25110 and 25120, capacity of 10 ml to 100 ml) were used stepwise in that order with the increase in the scale of cultivation. The growth rate was microscopically checked everyday. The medium was replaced with a fresh portion and the cell density was adjusted to the above-mentioned level at 3- or 4-day intervals. Every 10 days, PHA-P was added to the medium to a concentration of 0.1% and mitomycin-treated lymphocytes prepared as described in Example 1 were further added to attain a concentration of 2 to $8 \times 10^5$ cells/ml. In this way, the human cytotoxic T-cell clone could be cultured for a prolonged period of time and a sufficient number of cells could be obtained for diagnostic purposes.

## EXAMPLE 4

Specificity of human cytotoxic T-cell clone TA-NB-2

The 300 T-cell clones obtained in Example 1 were assayed for cytotoxic activity against hepatocytes from patients with type non-A non-B hepatitis and from patients with type B hepatitis by the cytotoxic activity assay method mentioned in Example 2. As a result, three cytotoxic T-cell clones (each capable of being cultured, as shown in Example 3, and thus proliferable artificially without limitation, hence differing in nature from cytotoxic T-cells occurring in vivo) were found to be specific to hepatocytes from patients with type non-A non-B hepatitis. They were designated TA-NB-1, TA-NB-2 and TA-NB-3, respectively. The specificity of each clone to hepatocytes from patients with type non-A non-B hepatitis is shown below in Table 1.

Table 1

| Cytotoxic T-cell Clone | Cytotoxic Activity against Hepatocytes from Patients with Type Non-A Non-B Hepatitis | Cytotoxic Activity against Hepatocytes from Patients with Type B Hepatitis |
|---|---|---|
| | (%) | (%) |
| TA-NB-1 | 33.0 | 0 |
| TA-NB-2 | 46.2 | 0 |
| TA-NB-3 | 38.5 | 3.5 |

Of these, the clone TA-NB-2, which had particularly excellent proliferability, was selected and assayed for cytotoxic activity against hepatocytes from 15 patient with type non-A non-B hepatitis. The results obtained are shown in Table 2 below. The human cytotoxic T-cell clone TA-NB-2 has been deposited, since August 25, 1988, with the Fermentation Research Institute, Agency of Industrial Science and Technology· under deposit number FERM P-10235 (after conversion to the Budapest Treaty route, FERM BP-2571).

Table 2

| Cytotoxic Activity of TA-NB-2 Against Hepatocytes from Patients with Type Non-A Non-B Hepatitis | | |
|---|---|---|
| Hepatocytes from Patients with Type Non-A Non-B Hepatitis | Disease | Cytotoxic Activity |
| | | (%) |
| 1 | CH (Note 1) | 46.2 |
| 2 | CH | 51.4 |
| 3 | CH | 42.3 |
| 4 | LC (Note 2) | 25.2 |
| 5 | LC | 24.4 |
| 6 | CH | 36.3 |
| 7 | CH | 14.2 |
| 8 | LC | 48.0 |
| 9 | CH | 35.0 |
| 10 | CH | 37.6 |
| 11 | CH | 35.3 |
| 12 | CH | 43.9 |
| 13 | CH | 38.7 |
| 14 | CH | 23.2 |
| 15 | CH | 55.7 |

Note 1: CH = Chronic hepatitis
Note 2: LC = Liver cirrhosis

The data shown in Table 2 demonstrate that TA-NB-2 showed cytotoxic activity against hepatocytes from all 15 patients with type non-A non-B hepatitis.

COMPARATIVE EXAMPLE 1

Specificity of human cytotoxic T-cell clone TA-NB-2

The data on the cytotoxic activity of TA-NB-2 as obtained against hepatocytes from 9 patients with type

7

EP 0 362 755 A2

B hepatitis are shown in Table 3 below.

Table 3

| Cytotoxic Activity of TA-NB-2 Against Hepatocytes from Patients with Type B Hepatitis | | |
|---|---|---|
| Hepatocytes from Patients with Type B Hepatitis | Disease | Cytotoxic Activity (%) |
| 1 | CH | 0 |
| 2 | CH | 0 |
| 3 | CH | 0 |
| 4 | CH | 1.4 |
| 5 | CH | 10.0 |
| 6 | AH (Note 3) | 2.4 |
| 7 | AH | 9.5 |
| 8 | CH | 10.9 |
| 9 | CH | 0.3 |

Note 3: AH = Acute hepatitis

The data shown in Table 3 demonstrate that TA-NB-2 did not show any significant (12% or more) cytotoxic activity against hepatocytes from all the nine patients with type B hepatitis.

From the data shown in Table 2 and Table 3, it is apparent that TA-NB-2 has cytotoxic activity which is specific to hepatocytes from patients with type non-A non-B hepatitis.

Phenotype analysis of the human cytotoxic T-cell clone TA-NB-2 was performed by immunofluorescence and flow cytometry using various commercially available monoclonal antibodies. The results thus obtained are shown in Table 4 below. The results shown in Table 4 demonstrate that TA-NB-2 has the CD3 and CD8 antigens, which are markers characteristic of cytotoxic T-cells, and a human T-cell antigen receptor composed of the $\alpha$ and $\beta$ chains on the cell surface. Since it is Leu-19 antigen positive, this clone is considered to be a T-cell clone making antigen recognition independently of any major histocompatibility complex. The techniques of fluorescence and flow cytometry are described in detail in a monograph such as Selected Methods in Cellular Immunology, edited by Barbara B. Mishell and Stanley M. Shiigi, W.H. Freeman, San Francisco, 1980; Japanese edition translated by Katsuyuki Imai et al, Rikogakusha, Tokyo, 1982, pages 273-278.

Table 4

| Cell Surface Antigens of Human Cytotoxic T-Cell Clone | | |
|---|---|---|
| | Cell Surface Antigen | Result |
| CD3 | T-cell antigen receptor | + |
| CD4 | Human T helper/inducer cell antigen | - |
| CD8 | Human T cytotoxic/suppressor cell antigen | + |
| CD16 | Fc receptor | - |
| Leu-19 | | + (weak) |
| TCR-1 | Human T-cell antigen receptor, $\alpha/\beta$ heterodimer | + |
| Ti-$\gamma$A | Human T-cell antigen receptor, $\gamma$ chain | - |
| TCR$\delta$1 | Human T-cell antigen receptor, $\delta$ chain | - |

EXAMPLE 5

8

EP 0 362 755 A2

Antibody against human cytotoxic T-cell clone TA-NB-2

TA-NB-2 cells were proliferated by the method described in Example 3 and $2 \times 10^7$ cells were obtained. These cells were suspended in 1 ml of RPMI 1640 medium. The suspension was thoroughly mixed with 1 ml of complete Freund's adjuvant (Difco, U.S.A.) to give 2 ml of an emulsion. The emulsion was subcutaneously injected into each of 10 BALB/c mice at a dose of 0.2 ml for the first immunization. Two weeks later, a second immunization was performed in the same manner except that incomplete Freund's adjuvant (Difco, U.S.A.) was used in lieu of complete Freund's adjuvant. After an additional two weeks, $1 \times 10^7$ TA-NB-2 cells were suspended in RPMI 1640 medium and the suspension was intraperitoneally given to five of the mice at a dose of 0.2 ml for the final immunization. Three days later, blood was collected from the thus-immunized mice and 2 ml of an antiserum was obtained. Immunofluorescence and flow cytometry confirmed that this antiserum could distinctly stain TA-NB-2 cells even when diluted 80-fold with 10 mM phosphate buffer (pH 7.2). The techniques of immunofluorescence and flow cytometry are described in detail in monographs such as Selected Methods in Cellular Immunology, Japanese edition, translated by Katsuyuki Imai et al, Rikogakusha, Tokyo, 1982, pages 273-278. Briefly $1 \times 10^6$ TA-NB-2 cells to be stained were suspended in 100 $\mu$l of RPMI medium supplemented with 10% fetal calf serum, 10 $\mu$l of an antibody diluting was added to the suspension with ice cooling, and the mixture was allowed to stand for 1 hour (in a 15-ml tube). 10 ml of the above medium was added thereto and, after thorough blending, the mixture was centrifuged (800 rpm, 10 minutes). The supernatant was removed, the above medium was added and the same procedure was performed. To the cell pellet was added 100 $\mu$l of the above medium and, after blending, 10 $\mu$l of 1 mg/ml Fluorescein conjugated anti-mouse Ig (Cappel, U.S.A.) was added, and the resulting mixture was allowed to stand on ice for 1 hour. Then, 10 ml of the medium was added and, after thorough blending, the mixture was centrifuged. The supernatant was removed and the same procedure was followed again and then 100 $\mu$l of the medium was added. After thorough blending, the resulting mixture was used as a sample. The sample was observed under a fluorescence microscope and whether fluorescence could be detected on the cell membrane was judged.

As mentioned above, an antiserum was readily obtained by immunizing mice with TA-NB-2 cells. The antiserum, however, reacted with the human T lymphoma cell line CEM as well. It was thus found that the antiserum contained not only the desired antibody against an antigen-binding site of T-cell receptor of the cytotoxic T-cell clone TA-NB-2 but also antibodies to membrane antigens common to human T-cells. For the removal of such antibodies, the antiserum was repeatedly subjected to an absorption procedure. Thus, $1 \times 10^8$ CEM cells were suspended in 2 ml of a 20-fold dilution of the antiserum and, after 1 hour of reaction on ice, the suspension was centrifuged (800 rpm, 10 minutes) to give a supernatant. The procedure comprising adding CEM cells to the supernatant and allowing the mixture to stand for absorption was repeated five times in all. Furthermore, a similar procedure comprising adding $3 \times 10^7$ peripheral blood lymphocytes from a healthy donor for absorption was repeated five times. In this way, an absorption-treated antiserum (antiidiotypic antiserum) was obtained.

Upon investigation by immunofluorescence and flow cytometry, this antiidiotypic antiserum strongly reacted with TA-NB-2 cells whereas the reactivity to CEM cells or to peripheral blood lymphocytes from healthy donors was scarcely detected.

EXAMPLE 6

Diagnosis of type non-A non B hepatitis using antiidiotypic antiserum

The reactivity of the antiidiotypic antiserum obtained in Example 5 for lymphocytes from patients with type non-A non-B hepatitis was compared with the reactivity of this antiserum for lymphocytes from healthy donors using flow cytometry. The techniques of flow cytometry are described in detail in monographs such as Flow Cytometry, Techniques and Practice, edited by Kazuo Ohta, Kanishobo, Tokyo, 1984. The results obtained are illustrated in Fig. 1. As shown in Fig. 1, the antiidiotypic antiserum according to the invention strongly reacted with lymphocytes from patients with type non-A non-B hepatitis in comparison with lymphocytes from healthy donors.

9

Thus it may be said that the blood of patients with type non-A non-B hepatitis contains an increased number of TA-NB-2 cell-like cytotoxic T-cells specific to hepatocytes from type non-A non-B hepatitis patients.

As mentioned above, the diagnosis of type non-A non-B hepatitis can be made using the antiidiotypic antiserum according to the invention.


EXAMPLE 7


Production of antiidiotypic monoclonal antibody


(1) Hybridoma Production

A mouse was immunized with the human cytotoxic T-cell clone TA-NB-2 by the method described in detail in Example 5. The spleen was excised from the mouse and minced in Hanks' balanced salt solution (Nissui, Japan). The splenocyte suspension thus obtained was centrifuged at 800 rpm for 10 minutes. The supernatant was discarded. RPMI 1640 medium (100 ml) was added to the cell pellet and splenocytes were suspended therein at a concentration of $5 \times 10^6$ cells/ml.

The splenocyte suspension was mixed with a mouse P3U1 myeloma cell suspension in 100 ml of RPMI 1640 medium (concentration $5 \times 10^6$ cells/ml) and the mixture was centrifuged at 800 rpm for 10 minutes. The supernatant was removed, 2 ml of 50% polyethylene glycol (PEG 4000, Merck, West Germany)-RPMI 1640 medium was added to the cell pellet. The resulting cell suspension was allowed to stand at 37°C for 7 minutes for cell fusion. Then, 100 ml of RPMI 1640 medium was slowly added to this suspension, the resulting mixture was centrifuged at 800 rpm for 10 minutes, the supernatant was removed, and 250 ml of RPMI medium supplemented with 20% fetal calf serum was added to the cell pellet to give a uniform cell suspension. This suspension was distributed in 100-$\mu$l portions into wells of several 96-well plates (Costar, U.S.A.) and incubated overnight at 37°C in an atmosphere of 5% $CO_2$. Then, 100 $\mu$l of HAT medium (cf. Hybridoma Techniques and Monoclonal Antibodies, edited by Takeshi Watanabe, R&D Planning, 1982) was added to each well and incubation was continued at 37°C in a 5% $CO_2$ atmosphere. After 2 weeks, hybridoma growth was evident and about 300 hybridoma strains were obtained. The above cell fusion procedure was repeated until a total of about 2,000 hybridoma strains were obtained.


(2) Screening for Antiidiotypic Monoclonal Antibody-producing Hybridoma Strains

The 2,000 hybridoma culture supernatants obtained were screened for specificity to TA-NB-2 and to other human cytotoxic T-cell clones (with known specificity; known to be not associated with type non-A non-B hepatitis virus) by the immunofluorescence method described in detail in Example 5. The hybridoma culture supernatants were each used as such in lieu of the diluted antibody solution used in Example 5. As a result, a hybridoma strain, d16, producing a monoclonal antibody specifically reactive with TA-NB-2 was obtained. The reactivity of this monoclonal antibody d16 to TA-NB-2 is shown in Fig. 2 in comparison with the reactivity thereof for another cytotoxic T-cell clone, namely the clone 51 [deposited with the Public Health Laboratory Service Centre for Applied Microbiology and Research (Great Britain) under the deposit number 85082201]. The specific reactivity of the monoclonal antibody d16 for TA-NB-2 is evident.

While the invention has been described in detail and with reference to specific embodiments thereof, it will be apparent to one skilled in the art that various changes and modifications can be made therein without departing from the spirit and scope thereof.


**Claims**

1. A human cytotoxic T-cell clone with significant cytotoxicity against hepatocytes from patients with type non-A non-B virus hepatitis but not against hepatocytes from patients with type B virus hepatitis.

2. An antibody to the human cytotoxic T-cell clone of Claim 1.

3. A method of diagnosing type non-A non-B hepatitis which comprises mixing the human cytotoxic T-cell clone of Claim 1 with a human body fluid sample and determining reactivity.

4. A method of diagnosing type non-A non-B hepatitis which comprises contacting the antibody of Claim 2 immunologically with lymphocytes in a human body fluid sample and determining those reactive with said antibody.

11

Fig. 1

Fig. 2